(19) Europäisches Patentamt
European Patent Office
Office européen des brevets

(11) Publication number : 0 471 564 A2

(12) # EUROPEAN PATENT APPLICATION

(21) Application number : 91307500.8

(22) Date of filing : 14.08.91

(51) Int. Cl.$^5$ : **C12N 15/78,** C12N 15/32, A01N 63/02, C12N 1/21, // (C12N1/21, C12R1:39, 1:38)

(30) Priority : 16.08.90 US 568440

(43) Date of publication of application : 19.02.92 Bulletin 92/08

(84) Designated Contracting States : AT BE CH DE DK ES FR GB GR IT LI LU NL SE

(71) Applicant : Mycogen Corporation
5451 Oberlin Drive
San Diego, CA 92121 (US)

(72) Inventor : Wilcox, Edward R.
5 Lopa Court
North Potomac, MD 20878 (US)
Inventor : Wong, Sui-Yin
7116 Cather Court
San Diego, California 92122 (US)

(74) Representative : Perry, Robert Edward et al
GILL JENNINGS & EVERY 53-64 Chancery Lane
London WC2A 1HN (GB)

(54) **Translational enhancement in pseudomonas.**

(57) A DNA sequence coding for an RNA translational enhancer, e.g. in Pseudomonas, has the sequence TTAATCTAC. This enhancer sequence can be incorporated into a suitable vector which can be used to transform a Pseudomonas, e.g. Pseudomonas fluorescens host. The vector may contain a gene encoding a useful protein, e.g. a Bacillus thuringiensis δ-endotoxin.

EP 0 471 564 A2

## Background of the Invention

There are several published methods for enhancing translation in prokaryotic microorganisms. The first observations were that of Shine, J. and L. Dalgarno (1974) Proc. Natl. Acad. Sci. USA 71:1342-1346, who showed that a short nucleotide sequence is required for efficient message translation. The so-called Shine-Dalgarno sequence (AGGAGGU) is complementary to RNA close to the 3′ end of the 16S ribosome. The optimal distance between the last base of the Shine-Dalgarno sequence and the start codon on the mRNA is typically 7-8 bp (Tessier, L et al. [1984] Nucl. Acids. Res. 12:7663-7675).

In a specialized ribosome translation system, described by AS. Hui and H.A DeBoer (1987) Proc. Natl. Acad. Sci. USA 84:4762-4766, the anti-Shine-Dalgarno sequence of the 16S ribosome has been changed to complement an altered Shine-Dalgarno in front of a human growth hormone gene. This system produces trp promoted human growth hormone at 25-30% of the newly synthesized proteins, after induction of the modified 16S ribosome.

A translational enhancer has been described from tobacco mosaic virus (Gallie, D.R., and C.I. Kado [1989] Proc. Natl. Acad. Sci. USA 86:129-132). In this instance, translational enhancement works in both eukaryotes and prokaryotes. The omega sequence, consisting of 68 bases or less of the 5′ untranslated leader of tobacco mosaic virus RNA (Gallie, D.R. et al. [1988] Nucl. Acids Res. 16:883-893), is able to function in place of a Shine-Dalgarno sequence even though there is no homology to the 3′ end of the 16S ribosome.

The phage T7 gene 10 leader sequence is also capable of enhancing translation (Olins, P.O. et al. [1988] Gene 73:227-235). This enhancer sequence is the probable basis for the observed ability of late T7 mRNA molecules to discriminate against phage T7 0.3 mRNA molecules (Strome, S., and E.T. Young [1980] J. Mol. Biol. 136:417-432; Strome, S., and E.T. Young [1980] J. Mol. Biol. 136:433-450).

The subject invention concerns translational enhancement in Pseudomonas. This enhancement is conferred by cloning the disclosed enhancer sequence into the 5′ untranslated portion of heterologously transcribed mRNA.

## Brief Summary of the Invention

The subject invention concerns a DNA sequence coding for an RNA translational enhancer in Pseudomonas. The DNA sequence is as follows:

5′TTAATCTAC 3′

The subject Pseudomonas enhancer sequence can be incorporated into a suitable vector, e.g., a plasmid, which can be used to transform Pseudomonas fluorescens or other Pseudomonas sp. host. The transformed host would then express higher amounts of a desired protein. For example, a plasmid can be constructed to comprise the enhancer sequence of the subject invention along with a promoter and a gene encoding a δ-endotoxin which is active against various pests. Examples of δ-endotoxins are those produced by various strains of Bacillus thuringiensis microbes.

The vector will have a DNA transcriptional promoter and the invention DNA enhancer coding sequence coding for an RNA translational enhancer. The DNA enhancer coding sequence in the vector is subject to the transcriptional promoter. A foreign DNA gene encoding a desired protein is engineered in the vector so that it is subject to the transcriptional promoter. After transcription of the foreign DNA gene and the DNA enhancer coding sequence to their RNA variants, translation of the RNA variant of the foreign DNA gene will be subject to the control of the RNA variant of the enhancer coding sequence of the invention.

The RNA translational enhancer is of the non-coding type.

## Brief Description of the Drawings

**Figure 1** - Restriction map of plasmid pMYC1186B.

**Figure 2** - Restriction map of plasmid pMYC2003.

**Figure 3** - Restriction map of plasmid pMYC2004.

**Figure 4** - Graph of toxin production from plasmid pMYC2003 and plasmid pMYC2004.

**Figure 5** - Graph comparison of toxin production by Pseudomonas fluorescens host MR820 transformed with a plasmid comprising the translational enhancer of the invention as compared to Pseudomonas fluorescens host MR818 which does not contain the translational enhancer.

## Detailed Description of the Invention

The subject invention can be used to enhance the expression of any gene in Pseudomonas fluorescens

or other Pseudomonas sp. Examples of genes are those which can be used to express toxins useful for agricultural purposes. Many of these genes are found in a variety of strains of Bacillus thuringiensis. Such genes generally encode δ-endotoxins which can be used to control a variety of agricultural pests, notable among which are lepidopteran, dipteran, and coleopteran insects, as well as nematode pests.

A wide variety of ways are available for introducing the B.t. gene expressing the toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA. The initiation and translational termination region will involve stop codon(s) and a transcriptional terminator region.

In the direction of transcription, namely in the 5′ to 3′ direction of the coding or sense sequence, the construct will involve the transcriptional regulatory region, if any, and the promoter, where the regulatory region may be either 5′ or 3′ of the promoter, the ribosomal binding site, the initiation codon, the structural gene having an open reading frame in phase with the initiation codon, the stop codon(s) and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototropy to an auxotrophic host, or the like. Preferably, complementation is employed, so that the modified host may not only be selected, but may also be competitie in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may electively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the Tac promoter, the naturally-occurring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome,

an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present.

Treatment of the microbial cell, e.g., a Pseudomonas fluorescens microbe containing the B.t. toxin gene, can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Bouin's fixative and Helly's finative (See: Humason, Gretchen L., Animal Tissue Techniques, W.H. Freeman and Company, 1967); or a combination of physical (heat) and chemical agents that preserve and prolong the activity of the toxin produced in the cell when the cell is dispersed into the environment of a target pest, or is administered to a host animal. Examples of physical means are short wavelength radiation such as gamma-radiation and X-radiation, freezing, UV irradiation, lyophilization, and the like.

The cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the B.t. insecticidal gene may be grown in any convenient nutrient medium, where the DNA construct provides a selective advantage, providing for a selective medium so that substantially all or all of the cells retain the B.t. gene. These cells may then be harvested in accordance with conventional ways. Alternatively, the cells can be treated prior to harvesting.

The B.t. cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about $10^2$ to about $10^4$ cells/mg. These formulations will be administered at about 50 mg (liquid or dry) to 1 kg or more per hectare.

The formulations can be applied to the environment of the insect pest(s), e.g., plants, soil or water, by spraying, dusting, sprinkling, or the like.

The translational enhancer of the invention also can be engineered into a vector comprising a gene encoding a nematicidal protein. The procedures for engineering such a vector are as described above. B. thuringiensis microbes having nematicidal activity are known and available to the public. One such microbe is B. thuringiensis PS17, NRRL B-18243.

The group of diseases described generally as helminthiasis is due to infection of an animal host with parasitic worms known as helminths. Helminthiasis is a prevalent and serious economic problem in domesticated

animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaria, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris, Caenorhabditis, and Parascaris. Certain of these, such as Nematodirus, Cooperia, and Oesophagostomum, attack primarily the intestinal tract while others, such as Dictyocaulus are found in the lungs. Still other parasites may be located in other tissues and organs of the body.

The toxin expressed by the engineered P. fluorescens host is useful as a nematocide for the control of soil nematodes and plant parasites selected from the genera Bursaphalenchus, Criconemella, Ditylenchus, Globedera, Helicotylenchus, Heterodera, Melodiogyne, Pratylenchus, Radolpholus, Rotelynchus or Tylenchus.

Alternatively, because some plant parasitic nematodes are obligate parasites, genes coding for nematocidal B.t. toxins can be engineered into plant cells to yield nematode-resistant plants. The methodology for engineering plant cells is well established (cf. Nester, E.W., Gordon, M.P., Amasino, R.M. and Yanofsky, M.F. [1984] Ann. Rev. Plant Physiol. 35:387-399).

The nematicidal toxin can be administered orally in a unit dosage form such as a capsule, bolus or tablet, or as a liquid drench when used as an anthelmintic in mammals. The drench is normally a solution, suspension or dispersion of the active ingredient, usually in water, together with a suspending agents such as bentonite and a wetting agent or like excipient. Generally, the drenches also contain an antifoaming agent. Drench formulations generally contain from about 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or dicalcium phosphate.

When the active compound is to be administered via an animal feedstuff, it is intimately dispersed in the feed or used as a top dressing or in the form of pellets which may then be added to the finished feed or, optionally, fed separately. Alternatively, the nematicidal compound may be administered to animals parenterally, for example, by intralumial, intramuscular, intratracheal, or subcutaneous injection, in which event the active ingredient is dissolved or dispersed in a liquid carrier vehicle.

The following examples will serve to merely illustrate the invention and its use. These examples should not be construed as limiting the invention in ways not set out in the claims.

## Materials and Methods

Cloning and DNA manipulation techniques are described in Maniatis, T., E.F. Fritsch, and J. Sambrook (1982) Molecular Cloning, a Laboratory Manual. Cold Spring Harbor Laboratory Publishers, Cold Spring Harbor, New York.

Restriction enzymes and other DNA modifying enzymes were purchased from Boehringer Mannheim Biochemicals, Indianapolis, IN 46250. DNA synthesis of probes and sequencing primers was performed on an Applied Biosystems 380A oligonucleotide synthesizer using beta-cyanoester chemistry. The synthetic oligonucleotides were purified by passive diffusion from 7.5% polyacrylamide gels containing 8 M urea. The vector pTJS260 is available from Dr. D.R. Helinski at the University of Califomia, San Diego, La Jolla, CA 92023 (Schmidhauser, T.J. [1986] Ph.D. Thesis, UCSD).

## Example 1 - Construction of Plasmid pMYC1186B

Plasmid pMYC467 (NRRL B-18428) is the starting material. The toxin-encoding sequence of this plasmid was removed as an NdeI-8081 to NsiI-11816fragment and replaced with a cryIA(c)-like toxin gene (Hofte, H., and H.R. Witely [1989] Micro. Rev. 53:242-255; Adang, M.J. et al. [1985] Gene 36:289-300). Subsequent work was done to remove the streptomycin resistance genes from the pTJS260 vector. The SacI-214 to NotI-1674 region of pTJS260 was deleted and the blunt ends ligated, after treating them with T4 DNA polymerase in the presence of dNTPs.

## Example 2 - Construction of pMYC2003

The NsiI-1028 to BamHI-10537 fragment was removed from pMYC1186B on an agarose gel and the pMYC2003 plasmid constructed by ligation of the following synthetic sequence with the tac promoter of pKK223-2.

```
EcoRI/P.fluorescens
overhang/enhancer        S/D          Start of the toxin gene
  AATT TTAATCTAC TAGT AGGAGGT AACTT ATG GAT AAC AAT CCG AAC
       AATTAGATG ATCA TCCTCCA TTGAA TAC CTA TTG TTA GGC TTG

                    NsiI
                  overhang
    ATC AAT GAA TGCA
    TAG TTA CTT
```

The above synthetic DNA, along with the tac promoter (isolated as a BamHI to EcoRI fragment from pKK223-3, available from Pharmacia, Inc., Piscataway, NJ 08854), was ligated into pMYC1186B which had been cut with BamHI and NsiI and the resulting 14Kb fragment isolated from a TAE agarose gel.

Example 3 - Construction of pMYC2004

The pMYC2004 plasmid was constructed in the same manner as pMYC2003. The E. coli enhancer sequence (Olins, P., supra) was incorporated into pMYC2004. This new plasmid is identical to pMYC2003 except it has incorporated the following synthetic sequence (see below) and differs from pMYC2003 in the enhancer sequence it includes.

```
EcoRI      E. coli
overhang   enhancer        S/D          Start of the toxin gene
  AATT   TTAACTTTA TAGT AGGAGGT AACTT ATG GAT AAC AAT CCG AAC
         AATTGAAAT ATCA TCCTCCA TTGAA TAC CTA TTG TTA GGC TTG

                NsiI
              overhang
ATC AAT GAA TGCA
TAG TTA CTT
```

Toxin production was compared in an experiment using the above two plasmids, pMYC2003 and pMYC2004 , in P. fluorescens. The data show more rapid toxin production (see Figure 4) when the P. fluorescens enhancer is used instead of the E. coli specific enhancer.

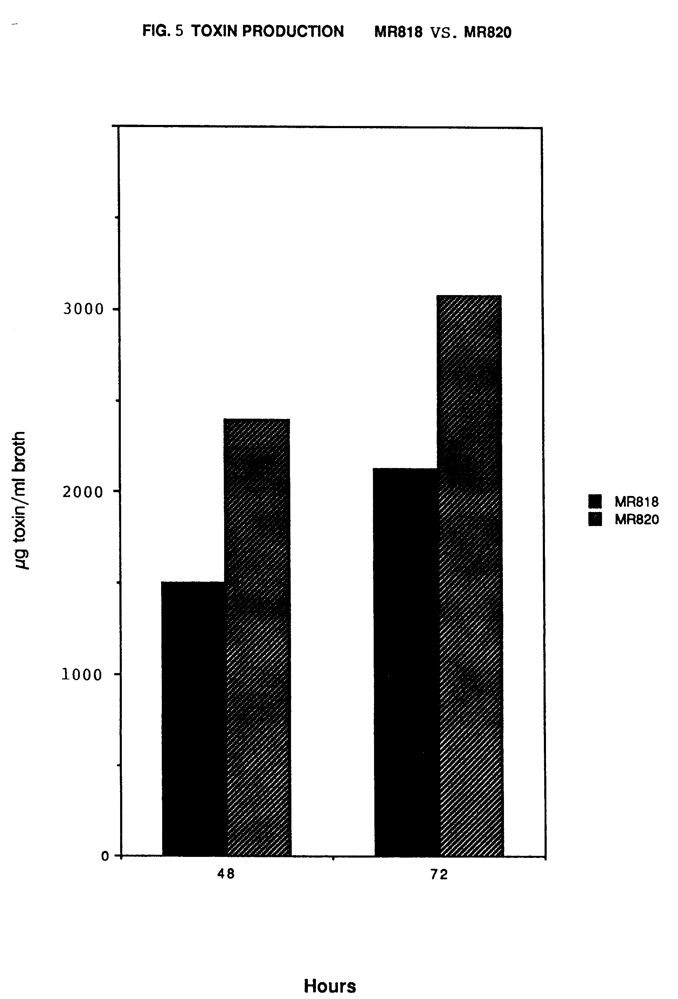

Another plasmid, pMYC1197, which is identical to pMYC2003 except that the NdeI-6507 to SacI8964 region is replaced with the NdeI to SacI fragment of cryIA(b), was transformed into P. fluorescens to give the strain designated MR820. See Seq. ID No. 1. As shown in Figure 5, this strain gives a very high, rapid yield of δ-endotoxin.

Example 4 - Toxin Production from pMYC2003 and pMYC2004

The two enhancer-containing plasmids, pMYC2003 and pMYC2004, were compared in a lactose inducible strain of P. fluorescens. A control plasmid (pTJS260) containing no toxin gene was also included for the growth comparison properties of the two plasmids. Cells were diluted 1:100 after overnight growth into LB and grown at 30°C until the optical density (OD 600 nm) was close to 0.30. The cells were then induced by the addition of IPTG (final concentration of 1 mM). Aliquots were harvested at the times indicated in the figure. Toxin concentration was determined using laser densitometry (LKB) after Coomassie staining of polyacrylamide gels containing SDS. Prior to three hours, not enough toxin was detected for scanning by laser densitometry and are not included with the figure. At three hours post-induction, P. fluorescens cells containing pMYC2003 produced 46% more toxin than cells containing pMYC2004. At 5 hours, there was 36% more toxin and at seven hours, 34% more toxin. By 20 hours post induction, there was no significant difference between the two samples. Figure 4 demonstrates that toxin accumulation is more rapid in the strain harboring the plasmid pMYC2003, containing the P. fluorescens translational enhancer, than the strain carrying plasmid pMYC2004, containing the E. coli translational enhancer. This more rapid protein accumulation provides a significant fermentation advantage.

Example 5 - Comparison of Toxin Production by *P.fluorescens* Hosts Designated MR820 and MR818

MR820 contains the enhancer sequence of the invention, whereas MR818 does not. Both cultures were grown in the following medium:

| | |
|---|---|
| Glycerol | 65 g/L |
| Na citrate•$2H_2O$ | 7.14 |
| HCT | 20 |
| Amberex 1003 | 20 |
| $NaNO_3$ | 5 |
| $(NH_4)_2SO_4$ | 2.3 |

32°C at 300 rpm.

These were 72 hour fermenattions with induction and supplementation taking place at 24 hours. These were induced with lactose and supplemented with the following:

| | |
|---|---|
| Amisoy | 20.0 g/L |
| $MgSO_4{\cdot}7H_2O$ | 0.4 |
| $K_2HPO_4$ | 1.6 |
| KCl | 1.6 |

Plate counts were done in L-broth agar with and without 30 μg/ml tetracycline at 24 hours.

MR818 3.6 x $10^{10}$ cfu/ml
MR820 2.4 x $10^{10}$ cfu/ml

Gelscan values at 48 and 72 hours:

| | 48 | 72 |
|---|---|---|
| MR818 | 1456 μg/ml | 2137 μg/ml |
| MR820 | 2271 μg/ml | 3083 μg/ml |

SEQUENCE LISTING

SEQ ID NO. 1
SEQUENCE TYPE: Nucleotide with corresponding protein
SEQUENCE LENGTH: 3462 base pairs
STRANDEDNESS: single
TOPOLOGY: linear
MOLECULE TYPE: genomic DNA
ORIGINAL SOURCE ORGANISM: Bacillus thuringiensis
PROPERTIES: Protozoan toxin

```
                                                                  Enhancer     S/D
                                                                   TTAA[illegible]CTACTAGTAGGAGGTAACTT

                    5                   10                  15                  20
Met Asp Asn Asn Pro Asn Ile Asn Glu Cys Ile Pro Tyr Asn Cys Leu Ser Asn Pro Glu
ATG GAT AAC AAT CCG AAC ATC AAT GAA TGC ATT CCT TAT AAT TGT TTA AGT AAC CCT GAA

                    25                  30                  35                  40
Val Glu Val Leu Gly Gly Glu Arg Ile Glu Thr Gly Tyr Thr Pro Ile Asp Ile Ser Leu
GTA GAA GTA TTA GGT GGA GAA AGA ATA GAA ACT GGT TAC ACC CCA ATC GAT ATT TCC TTG

                    45                  50                  55                  60
Ser Leu Thr Gln Phe Leu Leu Ser Glu Phe Val Pro Gly Ala Gly Phe Val Leu Gly Leu
TCG CTA ACG CAA TTT CTT TTG AGT GAA TTT GTT CCC GGT GCT GGA TTT GTG TTA GGA CTA

                    65                  70                  75                  80
Val Asp Ile Ile Trp Gly Ile Phe Gly Pro Ser Gln Trp Asp Ala Phe Leu Val Gln Ile
GTT GAT ATA ATA TGG GGA ATT TTT GGT CCC TCT CAA TGG GAC GCA TTT CTT GTA CAA ATT

                    85                  90                  95                  100
Glu Gln Leu Ile Asn Gln Arg Ile Glu Glu Phe Ala Arg Asn Gln Ala Ile Ser Arg Leu
GAA CAG TTA ATT AAC CAA AGA ATA GAA GAA TTC GCT AGG AAC CAA GCC ATT TCT AGA TTA

                    105                 110                 115                 120
Glu Gly Leu Ser Asn Leu Tyr Gln Ile Tyr Ala Glu Ser Phe Arg Glu Trp Glu Ala Asp
GAA GGA CTA AGC AAT CTT TAT CAA ATT TAC GCA GAA TCT TTT AGA GAG TGG GAA GCA GAT

                    125                 130                 135                 140
Pro Thr Asn Pro Ala Leu Arg Glu Glu Met Arg Ile Gln Phe Asn Asp Met Asn Ser Ala
CCT ACT AAT CCA GCA TTA AGA GAA GAG ATG CGT ATT CAA TTC AAT GAC ATG AAC AGT GCC

                    145                 150                 155                 160
Leu Thr Thr Ala Ile Pro Leu Leu Ala Val Gln Asn Tyr Gln Val Pro Leu Leu Ser Val
CTT ACA ACC GCT ATT CCT CTT TTG GCA GTT CAA AAT TAT CAA GTT CCT CTT TTA TCA GTA

                    165                 170                 175                 180
Tyr Val Gln Ala Ala Asn Leu His Leu Ser Val Leu Arg Asp Val Ser Val Phe Gly Gln
TAT GTT CAA GCT GCA AAT TTA CAT TTA TCA GTT TTG AGA GAT GTT TCA GTG TTT GGA CAA

                    185                 190                 195                 200
Arg Trp Gly Phe Asp Ala Ala Thr Ile Asn Ser Arg Tyr Asn Asp Leu Thr Arg Leu Ile
AGG TGG GGA TTT GAT GCC GCG ACT ATC AAT AGT CGT TAT AAT GAT TTA ACT AGG CTT ATT

                    205                 210                 215                 220
Gly Asn Tyr Thr Asp Tyr Ala Val Arg Trp Tyr Asn Thr Gly Leu Glu Arg Val Trp Gly
GGC AAC TAT ACA GAT TAT GCT GTA CGC TGG TAC AAT ACG GGA TTA GAA CGT GTA TGG GGA

                    225                 230                 235                 240
Pro Asp Ser Arg Asp Trp Val Arg Tyr Asn Gln Phe Arg Arg Glu Leu Thr Leu Thr Val
CCG GAT TCT AGA GAT TGG GTA AGG TAT AAT CAA TTT AGA AGA GAA TTA ACA CTA ACT GTA

                    245                 250                 255                 260
Leu Asp Ile Val Ala Leu Phe Pro Asn Tyr Asp Ser Arg Arg Tyr Pro Ile Arg Thr Val
TTA GAT ATC GTT GCT CTG TTC CCG AAT TAT GAT AGT AGA AGA TAT CCA ATT CGA ACA GTT

                    265                 270                 275                 280
Ser Gln Leu Thr Arg Glu Ile Tyr Thr Asn Pro Val Leu Glu Asn Phe Asp Gly Ser Phe
TCC CAA TTA ACA AGA GAA ATT TAT ACA AAC CCA GTA TTA GAA AAT TTT GAT GGT AGT TTT

                    285                 290                 295                 300
Arg Gly Ser Ala Gln Gly Ile Glu Arg Ser Ile Arg Ser Pro His Leu Met Asp Ile Leu
CGA GGC TCG GCT CAG GGC ATA GAA AGA AGT ATT AGG AGT CCA CAT TTG ATG GAT ATA CTT
```

305 310 315 320
Asn Ser Ile Thr Ile Tyr Thr Asp Ala His Arg Gly Tyr Tyr Tyr Trp Ser Gly His Gln
AAC AGT ATA ACC ATC TAT ACG GAT GCT CAT AGG GGT TAT TAT TAT TGG TCA GGG CAT CAA

325 330 335 340
Ile Met Ala Ser Pro Val Gly Phe Ser Gly Pro Glu Phe Thr Phe Pro Leu Tyr Gly Thr
ATA ATG GCT TCT CCT GTC GGT TTT TCG GGG CCA GAA TTC ACG TTT CCG CTA TAT GGA ACC

345 350 355 360
Met Gly Asn Ala Ala Pro Gln Gln Arg Ile Val Ala Gln Leu Gly Gln Gly Val Tyr Arg
ATG GGA AAT GCA GCT CCA CAA CAA CGT ATT GTT GCT CAA CTA GGT CAG GGC GTG TAT AGA

365 370 375 380
Thr Leu Ser Ser Thr Phe Tyr Arg Arg Pro Phe Asn Ile Gly Ile Asn Asn Gln Gln Leu
ACA TTA TCC TCT ACT TTT TAT AGA AGA CCT TTT AAT ATA GGG ATA AAT AAT CAA CAA CTA

385 390 395 400
Ser Val Leu Asp Gly Thr Glu Phe Ala Tyr Gly Thr Ser Ser Asn Leu Pro Ser Ala Val
TCT GTT CTT GAC GGG ACA GAA TTT GCT TAT GGA ACC TCC TCA AAT TTG CCA TCC GCT GTA

405 410 415 420
Tyr Arg Lys Ser Gly Thr Val Asp Ser Leu Asp Glu Ile Pro Pro Gln Asn Asn Asn Val
TAC AGA AAA AGC GGA ACG GTA GAT TCG CTG GAT GAA ATA CCA CCA CAG AAT AAC AAC GTG

425 430 435 440
Pro Pro Arg Gln Gly Phe Ser His Arg Leu Ser His Val Ser Met Phe Arg Ser Gly Ser
CCA CCT AGG CAA GGA TTT AGT CAT CGA TTA AGC CAT GTT TCA ATG TTT CGT TCA GGC TCT

445 450 455 460
Ser Ser Ser Val Ser Ile Ile Arg Ala Pro Met Phe Ser Trp Ile His Arg Ser Ala Glu
AGT AGT AGT GTA AGT ATA ATA AGA GCT CCT ATG TTC TCT TGG ATA CAT CGT AGT GCT GAA

465 470 475 480
Phe Asn Asn Ile Ile Pro Ser Ser Gln Ile Thr Gln Ile Pro Leu Thr Lys Ser Thr Asn
TTT AAT AAT ATA ATT CCT TCA TCA CAA ATT ACA CAA ATA CCT TTA ACA AAA TCT ACT AAT

485 490 495 500
Leu Gly Ser Gly Thr Ser Val Val Lys Gly Pro Gly Phe Thr Gly Gly Asp Ile Leu Arg
CTT GGC TCT GGA ACT TCT GTC GTT AAA GGA CCA GGA TTT ACA GGA GGA GAT ATT CTT CGA

505 510 515 520
Arg Thr Ser Pro Gly Gln Ile Ser Thr Leu Arg Val Asn Ile Thr Ala Pro Leu Ser Gln
AGA ACT TCA CCT GGC CAG ATT TCA ACC TTA AGA GTA AAT ATT ACT GCA CCA TTA TCA CAA

525 530 535 540
Arg Tyr Arg Val Arg Ile Arg Tyr Ala Ser Thr Thr Asn Leu Gln Phe His Thr Ser Ile
AGA TAT CGG GTA AGA ATT CGC TAC GCT TCT ACC ACA AAT TTA CAA TTC CAT ACA TCA ATT

545 550 555 560
Asp Gly Arg Pro Ile Asn Gln Gly Asn Phe Ser Ala Thr Met Ser Ser Gly Ser Asn Leu
GAC GGA AGA CCT ATT AAT CAG GGG AAT TTT TCA GCA ACT ATG AGT AGT GGG AGT AAT TTA

565 570 575 580
Gln Ser Gly Ser Phe Arg Thr Val Gly Phe Thr Thr Pro Phe Asn Phe Ser Asn Gly Ser
CAG TCC GGA AGC TTT AGG ACT GTA GGT TTT ACT ACT CCG TTT AAC TTT TCA AAT GGA TCA

585 590 595 600
Ser Val Phe Thr Leu Ser Ala His Val Phe Asn Ser Gly Asn Glu Val Tyr Ile Asp Arg
AGT GTA TTT ACG TTA AGT GCT CAT GTC TTC AAT TCA GGC AAT GAA GTT TAT ATA GAT CGA

605 610 615 620
Ile Glu Phe Val Pro Ala Glu Val Thr Phe Glu Ala Glu Tyr Asp Leu Glu Arg Ala Gln
ATT GAA TTT GTT CCG GCA GAA GTA ACC TTT GAG GCA GAA TAT GAT TTA GAA AGA GCA CAA

```
                    625                     630                     635                     640
Lys Ala Val Asn Glu Leu Phe Thr Ser Ser Asn Gln Ile Gly Leu Lys Thr Asp Val Thr
AAG GCG GTG AAT GAG CTG TTT ACT TCT TCC AAT CAA ATC GGG TTA AAA ACA GAT GTG ACG

                    645                     650                     655                     660
Asp Tyr His Ile Asp Gln Val Ser Asn Leu Val Glu Cys Leu Ser Asp Glu Phe Cys Leu
GAT TAT CAT ATT GAT CAA GTA TCC AAT TTA GTT GAG TGT TTA TCT GAT GAA TTT TGT CTG

                    665                     670                     675                     680
Asp Glu Lys Lys Glu Leu Ser Glu Lys Val Lys His Ala Lys Arg Leu Ser Asp Glu Arg
GAT GAA AAA AAA GAA TTG TCC GAG AAA GTC AAA CAT GCG AAG CGA CTT AGT GAT GAG CGG

                    685                     690                     695                     700
Asn Leu Leu Gln Asp Pro Asn Phe Arg Gly Ile Asn Arg Gln Leu Asp Arg Gly Trp Arg
AAT TTA CTT CAA GAT CCA AAC TTT AGA GGG ATC AAT AGA CAA CTA GAC CGT GGC TGG AGA

                    705                     710                     715                     720
Gly Ser Thr Asp Ile Thr Ile Gln Gly Gly Asp Asp Val Phe Lys Glu Asn Tyr Val Thr
GGA AGT ACG GAT ATT ACC ATC CAA GGA GGC GAT GAC GTA TTC AAA GAG AAT TAC GTT ACG

                    725                     730                     735                     740
Leu Leu Gly Thr Phe Asp Glu Cys Tyr Pro Thr Tyr Leu Tyr Gln Lys Ile Asp Glu Ser
CTA TTG GGT ACC TTT GAT GAG TGC TAT CCA ACG TAT TTA TAT CAA AAA ATA GAT GAG TCG

                    745                     750                     755                     760
Lys Leu Lys Ala Tyr Thr Arg Tyr Gln Leu Arg Gly Tyr Ile Glu Asp Ser Gln Asp Leu
AAA TTA AAA GCC TAT ACC CGT TAC CAA TTA AGA GGG TAT ATC GAA GAT AGT CAA GAC TTA

                    765                     770                     775                     780
Glu Ile Tyr Leu Ile Arg Tyr Asn Ala Lys His Glu Thr Val Asn Val Pro Gly Thr Gly
GAA ATC TAT TTA ATT CGC TAC AAT GCC AAA CAC GAA ACA GTA AAT GTG CCA GGT ACG GGT

                    785                     790                     795                     800
Ser Leu Trp Pro Leu Ser Ala Pro Ser Pro Ile Gly Lys Cys Ala His His Ser His His
TCC TTA TGG CCG CTT TCA GCC CCA AGT CCA ATC GGA AAA TGT GCC CAT CAT TCC CAT CAT

                    805                     810                     815                     820
Phe Ser Leu Asp Ile Asp Val Gly Cys Thr Asp Leu Asn Glu Asp Leu Gly Val Trp Val
TTC TCC TTG GAC ATT GAT GTT GGA TGT ACA GAC TTA AAT GAG GAC TTA GGT GTA TGG GTG

                    825                     830                     835                     840
Ile Phe Lys Ile Lys Thr Gln Asp Gly His Ala Arg Leu Gly Asn Leu Glu Phe Leu Glu
ATA TTC AAG ATT AAG ACG CAA GAT GGC CAT GCA AGA CTA GGA AAT CTA GAA TTT CTC GAA

                    845                     850                     855                     860
Glu Lys Pro Leu Val Gly Glu Ala Leu Ala Arg Val Lys Arg Ala Glu Lys Lys Trp Arg
GAG AAA CCA TTA GTA GGA GAA GCA CTA GCT CGT GTG AAA AGA GCG GAG AAA AAA TGG AGA

                    865                     870                     875                     880
Asp Lys Arg Glu Lys Leu Glu Trp Glu Thr Asn Ile Val Tyr Lys Glu Ala Lys Glu Ser
GAC AAA CGT GAA AAA TTG GAA TGG GAA ACA AAT ATT GTT TAT AAA GAG GCA AAA GAA TCT

                    885                     890                     895                     900
Val Asp Ala Leu Phe Val Asn Ser Gln Tyr Asp Arg Leu Gln Ala Asp Thr Asn Ile Ala
GTA GAT GCT TTA TTT GTA AAC TCT CAA TAT GAT AGA TTA CAA GCG GAT ACC AAC ATC GCG

                    905                     910                     915                     920
Met Ile His Ala Ala Asp Lys Arg Val His Ser Ile Arg Glu Ala Tyr Leu Pro Glu Leu
ATG ATT CAT GCG GCA GAT AAA CGC GTT CAT AGC ATT CGA GAA GCT TAT CTG CCT GAG CTG

                    925                     930                     935                     940
Ser Val Ile Pro Gly Val Asn Ala Ala Ile Phe Glu Glu Leu Glu Gly Arg Ile Phe Thr
TCT GTG ATT CCG GGT GTC AAT GCG GCT ATT TTT GAA GAA TTA GAA GGG CGT ATT TTC ACT
```

```
                 945                 950                 955                 960
Ala Phe Ser Leu Tyr Asp Ala Arg Asn Val Ile Lys Asn Gly Asp Phe Asn Asn Gly Leu
GCA TTC TCC CTA TAT GAT GCG AGA AAT GTC ATT AAA AAT GGT GAT TTT AAT AAT GGC TTA

                 965                 970                 975                 980
Ser Cys Trp Asn Val Lys Gly His Val Asp Val Glu Glu Gln Asn Asn His Arg Ser Val
TCC TGC TGG AAC GTG AAA GGG CAT GTA GAT GTA GAA GAA CAA AAC AAC CAC CGT TCG GTC

                 985                 990                 995                1000
Leu Val Val Pro Glu Trp Glu Ala Glu Val Ser Gln Glu Val Arg Val Cys Pro Gly Arg
CTT GTT GTT CCG GAA TGG GAA GCA GAA GTG TCA CAA GAA GTT CGT GTC TGT CCG GGT CGT

                1005                1010                1015                1020
Gly Tyr Ile Leu Arg Val Thr Ala Tyr Lys Glu Gly Tyr Gly Glu Gly Cys Val Thr Ile
GGC TAT ATC CTT CGT GTC ACA GCG TAC AAG GAG GGA TAT GGA GAA GGT TGC GTA ACC ATT

                1025                1030                1035                1040
His Glu Ile Glu Asn Asn Thr Asp Glu Leu Lys Phe Ser Asn Cys Val Glu Glu Glu Val
CAT GAG ATC GAG AAC AAT ACA GAC GAA CTG AAG TTT AGC AAC TGT GTA GAA GAG GAA GTA

                1045                1050                1055                1060
Tyr Pro Asn Asn Thr Val Thr Cys Asn Asp Tyr Thr Ala Thr Gln Glu Glu Tyr Glu Gly
TAT CCA AAC AAC ACG GTA ACG TGT AAT GAT TAT ACT GCG ACT CAA GAA GAA TAT GAG GGT

                1065                1070                1075                1080
Thr Tyr Thr Ser Arg Asn Arg Gly Tyr Asp Gly Ala Tyr Glu Ser Asn Ser Ser Val Pro
ACG TAC ACT TCT CGT AAT CGA GGA TAT GAC GGA GCC TAT GAA AGC AAT TCT TCT GTA CCA

                1085                1090                1095                1100
Ala Asp Tyr Ala Ser Ala Tyr Glu Glu Lys Ala Tyr Thr Asp Gly Arg Arg Asp Asn Pro
GCT GAT TAT GCA TCA GCC TAT GAA GAA AAA GCA TAT ACA GAT GGA CGA AGA GAC AAT CCT

                1105                1110                1115                1120
Cys Glu Ser Asn Arg Gly Tyr Gly Asp Tyr Thr Pro Leu Pro Ala Gly Tyr Val Thr Lys
TGT GAA TCT AAC AGA GGA TAT GGG GAT TAC ACA CCA CTA CCA GCT GGC TAT GTG ACA AAA

                1125                1130                1135                1140
Glu Leu Glu Tyr Phe Pro Glu Thr Asp Lys Val Trp Ile Glu Ile Gly Glu Thr Glu Gly
GAA TTA GAG TAC TTC CCA GAA ACC GAT AAG GTA TGG ATT GAG ATC GGA GAA ACG GAA GGA

                1145                1150
Thr Phe Ile Val Asp Ser Val Glu Leu Leu Leu Met Glu Glu
ACA TTC ATC GTG GAC AGC GTG GAA TTA CTT CTT ATG GAG GAA
```

## Claims

1. A translational enhancer for Pseudomonas having the sequence TTAATCTAC.

2. A recombinant DNA vector comprising:
   (a) a DNA transcriptional promoter and, operatively linked thereto and promotable thereby, both:
   (b) the DNA sequence TTAATCTAC; and
   (c) a foreign gene coding for a desired protein.

3. A vector according to claim 2, wherein the protein is a Bacillus thuringiensis.

4. A vector according to claim 3, wherein the $\delta$-endotoxin is toxic to insects selected from lepidoptera, diptera and coleoptera, or to nematodes.

5. A Pseudomonas strain hosting the DNA vector according to any of claims 2 to 4.

**6.** A strain according to claim 5, which is of Pseudomonas fluorescens, e.g. MR820.

**7.** A vector according to any of claims 2 to 4, which is a plasmid, e.g. plasmid pMYC2003.

**8.** Substantially intact, treated cells, having prolonged pesticidal activity when applied to the environment of a target pest, comprising an intracellular polypeptide toxic to the pest, in which the polypeptide is produced as a result of expression of a transformed Pseudomonas microbe containing a plasmid comprising a translational enhancer having the sequence TTAATCTAC.

**9.** Cells according to claim 8, which are Pseudomonas cells hosting a DNA vector according to claim 2, and in which the polypeptide is a result of expression of a Bacillus thuringiensis toxin gene.

**10.** Cells according to claim 8 or claim 9, wherein the Pseudomonas is Pseudomonas fluorescens.

**11.** Cells according to any of claims 8 to 10, which have been treated with iodine.

**12.** A process for preparing a protein, which comprises culturing a strain according to claim 5 or claim 6.

**13.** A hybrid gene having the nucleotide sequence shown in Seq. ID No. 1.

**14.** An insecticidal toxin having the amino-acid sequence shown in Seq. ID No. 1.

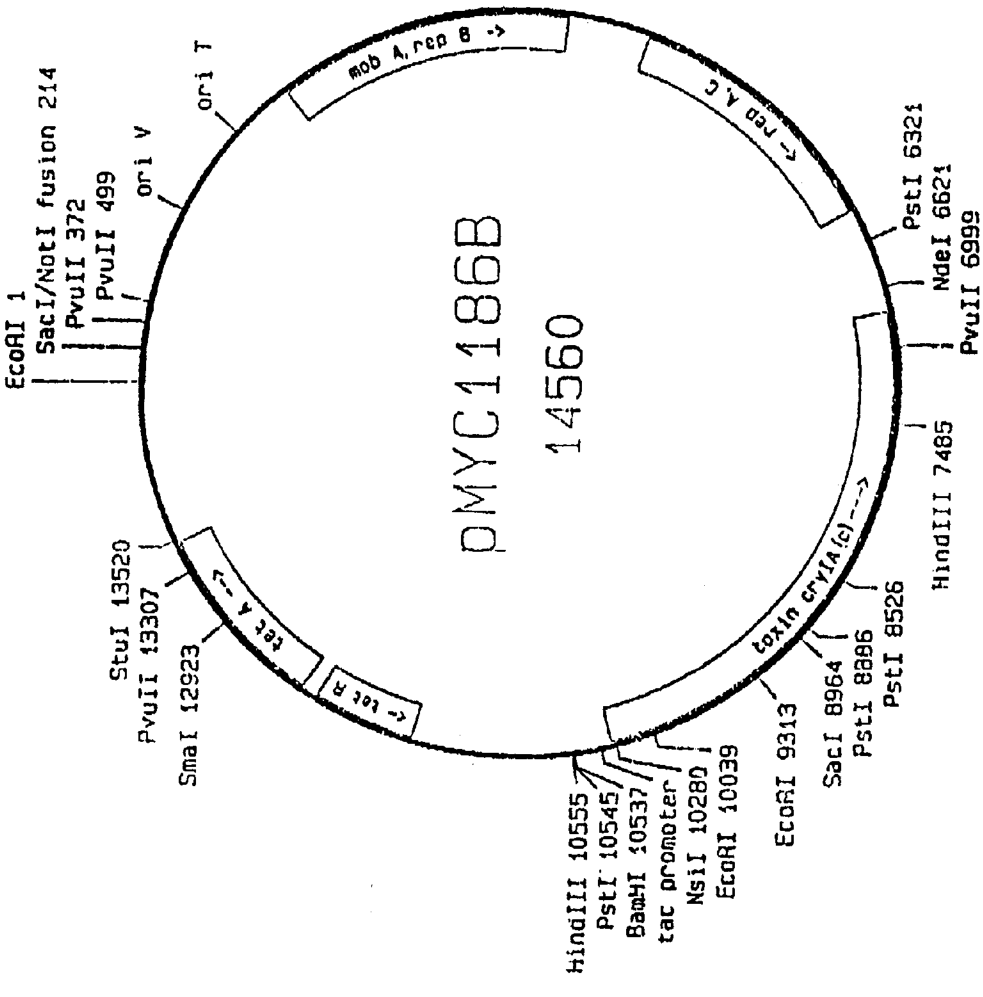
pMYC1186B
14560
mob A, rep B ->
<- rep A,C
toxin cryIA(c) --->
<-- tet A
<- tet R
ori T
ori V
EcoRI 1
SacI/NotI fusion 214
PvuII 372
PvuII 499
PstI 6321
NdeI 6621
PvuII 6999
HindIII 7485
PstI 8526
PstI 8886
SacI 8964
EcoRI 9313
EcoRI 10039
NsiI 10280
tac promoter
BamHI 10537
PstI 10545
HindIII 10555
SmaI 12923
PvuII 13307
StuI 13520

Figure 1

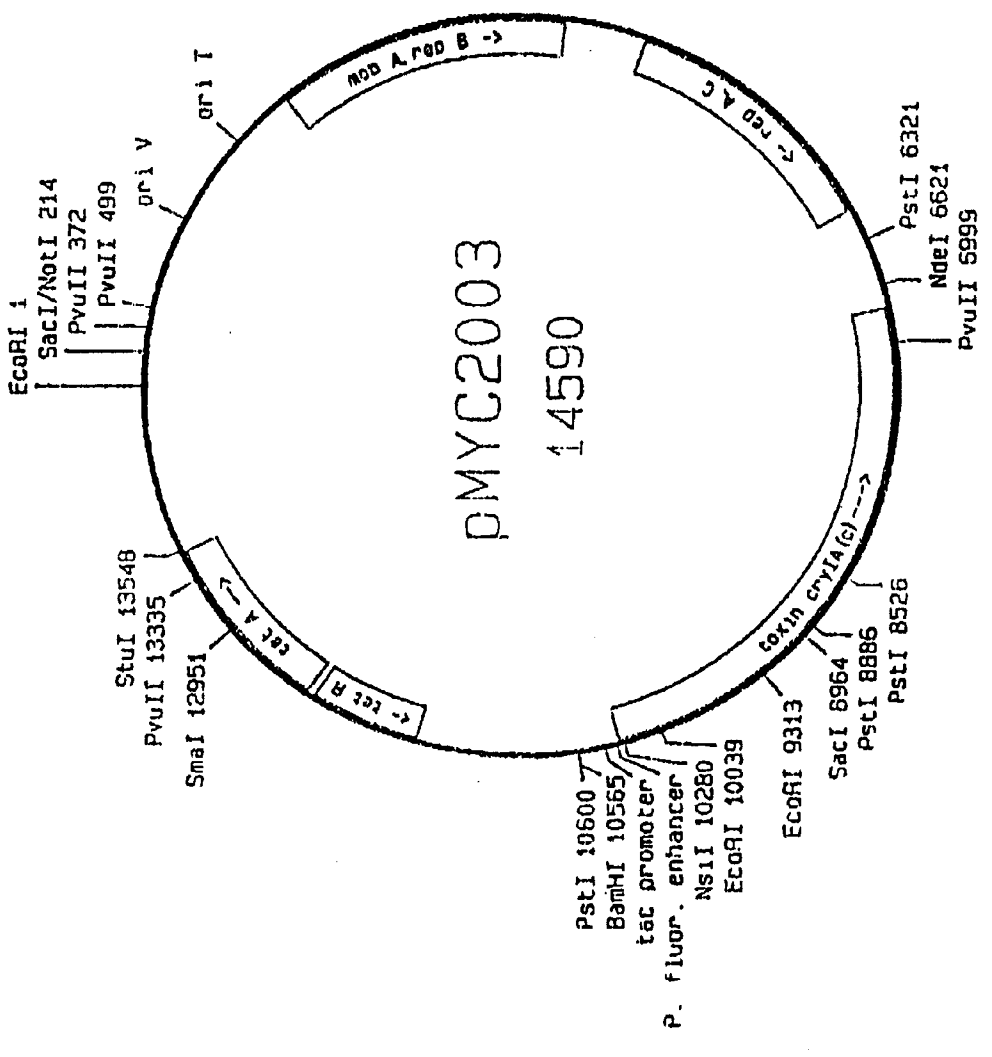
pMYC2003
14590
EcoRI 1
SacI/NotI 214
PvuII 372
PvuII 499
ori V
ori T
mob A, rep B ->
<- rep A C
PstI 6321
NdeI 6621
PvuII 6999
toxin cryIA(c)--->
PstI 8526
PstI 8886
SacI 8964
EcoRI 9313
EcoRI 10039
NsiI 10280
P. fluor. enhancer
tac promoter
BamHI 10565
PstI 10600
<- tet R
tet A <-
SmaI 12951
PvuII 13335
StuI 13548

Figure 2

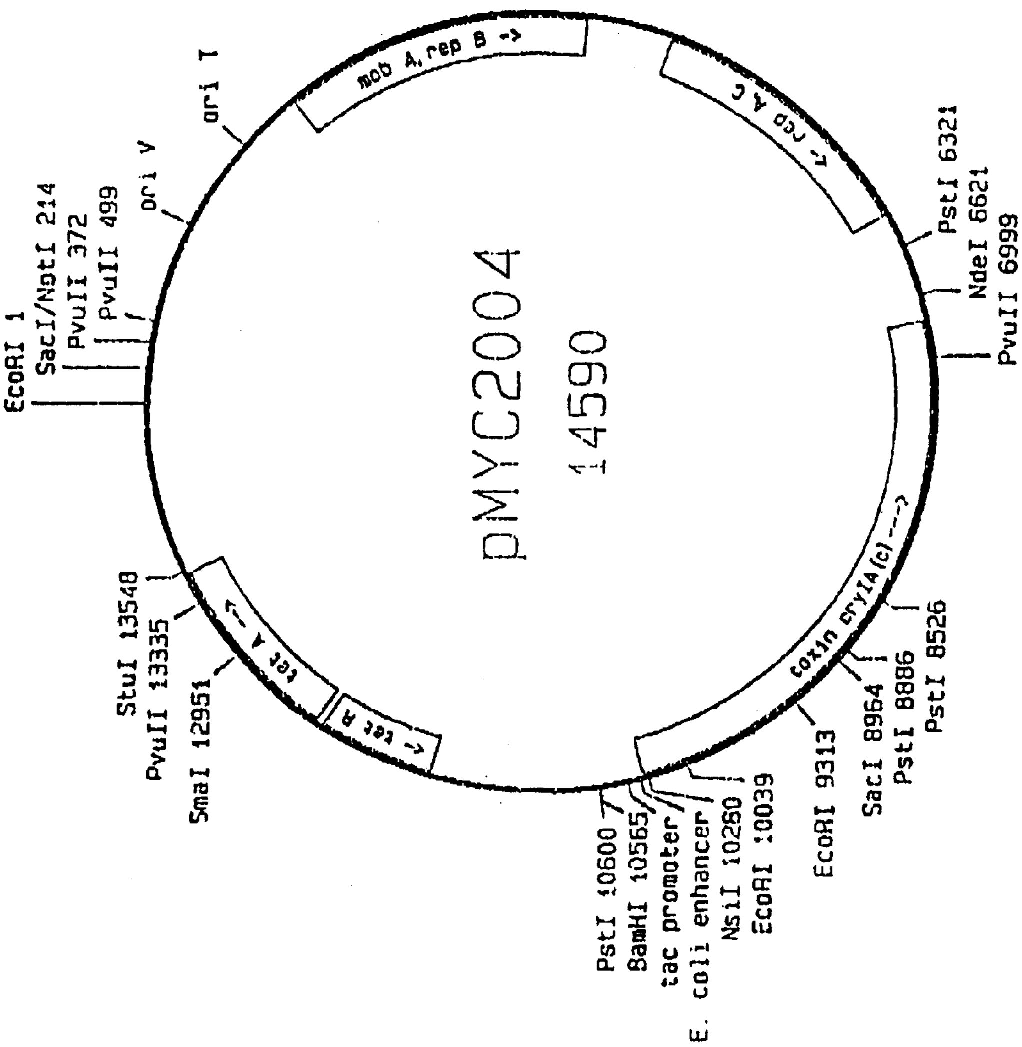
pMYC2004
14590
EcoRI 1
SacI/NotI 214
PvuII 372
PvuII 499
ori V
ori T
mob A, rep B ->
<- rep A C
PstI 6321
NdeI 6621
PvuII 6999
toxin cryIA(c) --->
PstI 8526
PstI 8886
SacI 8964
EcoRI 9313
EcoRI 10039
NsiI 10260
E. coli enhancer
tac promoter
BamHI 10565
PstI 10600
<- tet R
tet A <-
SmaI 12951
PvuII 13335
StuI 13548

Figure 3

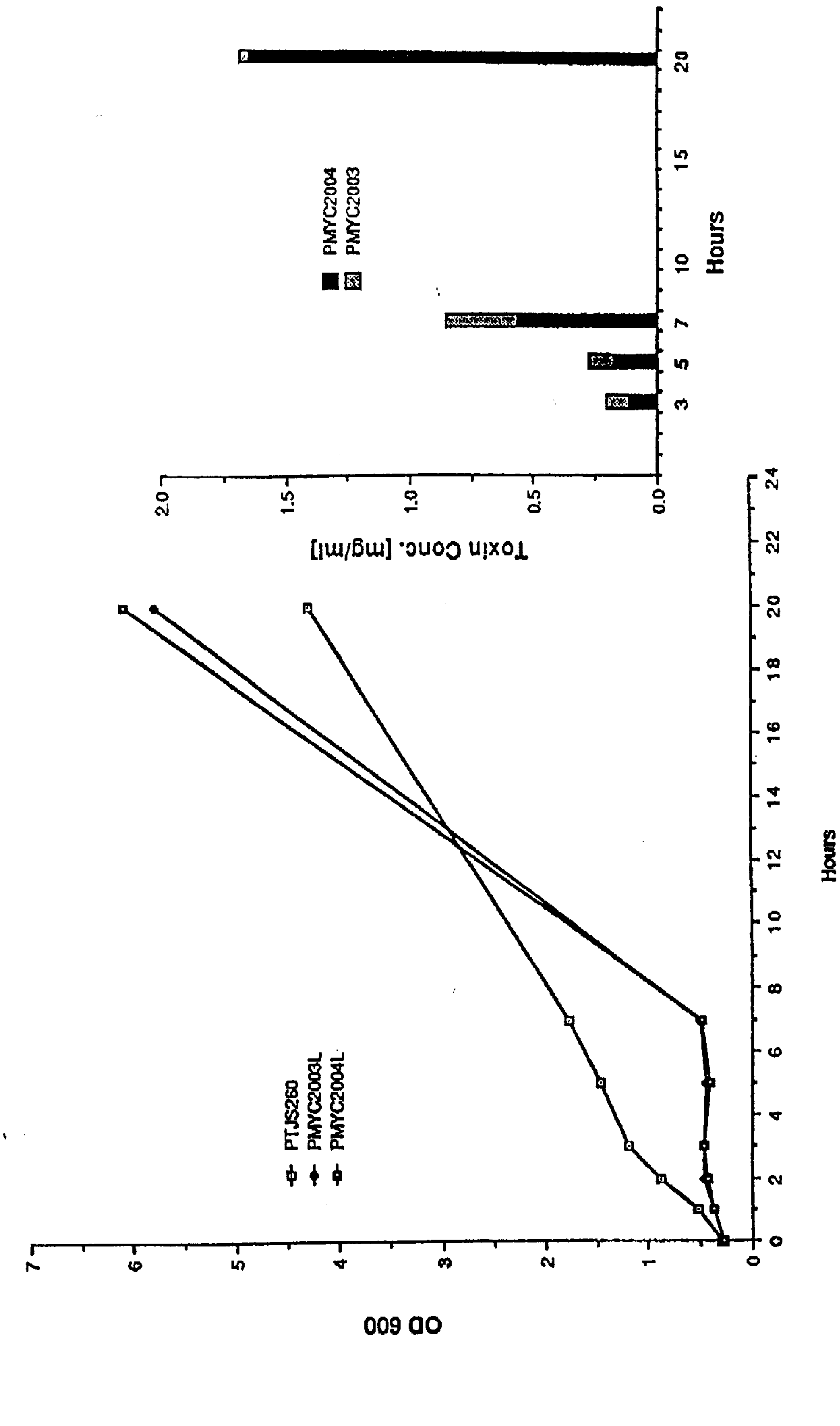
PMYC2004
PMYC2003
Toxin Conc. [mg/ml]
Hours
PTJS260
PMYC2003L
PMYC2004L
OD 600
Hours

Figure 4

FIG. 5 TOXIN PRODUCTION MR818 VS. MR820